# EUROPÄISCHE PATENTSCHRIFT

(11) **EP 1 235 522 B1**
(45) Veröffentlichungstag und Bekanntmachung des Hinweises auf die Patenterteilung: **30.05.2007**
(21) Anmeldenummer: 00965950.9
(22) Anmeldetag: 08.09.2000
(51) Int. Cl.: A61B 17/28

(54) **CHIRURGISCHES INSTRUMENT FÜR MINIMAL INVASIVE EINGRIFFE**
SURGICAL INSTRUMENT FOR MINIMALLY INVASIVE SURGICAL INTERVENTIONS
INSTRUMENT CHIRURGICAL POUR INTERVENTION MINIMALE INVASIVE

(30) Priorität: 09.09.1999 DE 19943018; 25.07.2000 DE 10036108
(43) Veröffentlichungstag der Anmeldung: 04.09.2002
(60) Teilanmeldung: 02013327.8
(73) Patentinhaber: Tuebingen Scientific Medical GmbH, 72074 Tuebingen (DE)
(72) Erfinder: SCHWARZ, Knut, M., 72072 Tübingen (DE); SCHURR, Marc, O., D-72074 Tübingen (DE); BUESZ, Gerhard, F., 72074 Tübingen (DE)
(74) Vertreter: TBK-Patent
(86) Internationale Anmeldenummer: PCT/EP2000/008802
(87) Internationale Veröffentlichungsnummer: WO 2001/017442

(56) Entgegenhaltungen:
- EP-A- 0 630 614
- WO-A-01/00095
- DE-A- 19 835 445
- DE-C- 19 647 761
- US-A- 5 281 220
- US-A- 5 549 637
- US-A- 5 827 323
- US-A- 5 836 960

## Beschreibung

Die vorliegende Erfindung betrifft ein chirurgisches Instrument insbesondere zur Verwendung bei minimal invasiven chirurgischen Techniken.

Die minimal invasive Chirurgie bedeutet für einen Patienten im Vergleich zur offenen Chirurgie hauptsächlich niedrigere Morbiditätsraten sowie kürzere Erholungszeiten und verursachen damit auch wesentlich geringere Vorgangs- bzw. Gesamtbehandlungskosten. Durch jüngste Entwicklungen auf diesem Gebiet konnten dabei Arbeitsgeräte bzw. Instrumente für die minimal invasive Chirurgie auch für komplexere Eingriffe etwa in der Herzchirurgie tauglich gemacht werden und helfen so mit, die Risiken und Belastungen für den Patienten auch bei solchen komplizierten Eingriffen zu reduzieren und Kosten für private sowie gesetzlichen Versicherungen zu senken. Das Einsatzspektrum der minimal invasiven Chirurgietechnik soll zukünftig jedoch nicht nur erweitert, sondern die hierfür verwendeten Instrumente trotz der immer weiter verfeinerten Chirurgietechniken zusätzlich preiswerter werden.

Aus der EP 0 636 010 ist eine chirurgische Vorrichtung dieser Gattung bekannt. Diese Vorrichtung besteht unter anderem aus einem rohr- oder schaftförmigen Gehäuse mit einer axial sich erstreckenden Schaftbohrung. In dieser Bohrung verläuft ein erstes längliches Bauteil mit einem proximalen und einem distalen Segment, welches teilweise aus einem elastischen vorgeformten (gekrümmten) Werkstoff besteht. Wenn das distale Ende nunmehr aus der Längsbohrung ausgefahren wird, nimmt dieses eine erste Form entsprechend der Vorformung an, während es für den Fall, dass es in die Bohrung zurückgezogen wird, eine zweite Form entsprechend der Form der Schaftbohrung annimmt.

Ferner ist ein zweites längliches Bauteil mit ebenfalls einem proximalen und einem distalen Segment vorgesehen, wobei das zweite Bauteil an das erste Bauteil im Parallelabstand zum ersten Bauteil angelenkt ist und von diesem gebogen wird, wenn sich das erste Bauteil von seiner ersten in seine zweite Form und umgekehrt ändert. Zusätzlich ist das zweite Bauteil um die Achse des ersten Bauteils relativ drehbar gehalten.

Am distalen Segment des zweiten länglichen Bauteils befindet sich ein Arbeitskopf oder Maulteil, welcher sich zusammen mit dem zweiten Bauteil bei dessen Biege- oder Krümmungsbewegung mitbewegt und vom zweiten Bauteil um die Achse des ersten Bauteils gedreht werden kann. Durch diese Ausbildung kann eine Bedienungsperson den Kopf der chirurgischen Vorrichtung beispielsweise einlang eines in einer Ebene gekrümmten Wegs bewegen und dann den Arbeitskopf in einer anderen Dimension, beispielsweise um die durch die Richtung des gekrümmten Wegs definierte Achse drehen.

Des weiteren ist aus der US-A-5 549 637 ein chirurgisches Instrument bekannt, welches dem vorliegenden Erfindungsgegenstand am nächsten kommt. Dieses bekannte Instrument hat einen Hohlschaft, an dessen einem, proximalen Ende eine Betätigungsvorrichtung und an dessen anderem, distalen Ende eine zum Schaft abwinkelbare Instrumentenspitze angeordnet ist. Ferner hat das Instrument einen Getriebemechanismus, welcher eine Bewegung der Betätigungsvorrichtung in eine Rotation der Instrumentenspitze unter einem ersten bestimmten Übersetzungsverhältnis zur Betätigungsbewegung transformiert. Eine weitere Bewegung der Betätigungsvorrichtung bewirkt eine Abwinklungsbewegung der Instrumentenspitze unabhängig zur Rotationsbewegung. Indessen ist die Betätigungsvorrichtung der US-A-5 549 637 so gestaltet, dass die beiden Bewegungen der Instrumentenspitze nur durch beidhändiges Betätigen der Betätigungsvorrichtung möglich sind, wodurch die Funktionalität des Instruments eingeschränkt wird.

Aus der zum vorliegenden Anmeldungsgegenstand nachveröffentlichten WO-A1-01/00095 ist ein chirurgisches Instrument bekannt mit einem Hohlschaft, an dessen einem, proximalen Ende eine Betätigungsvorrichtung mit Handhabe, die ein knopfartiges Griffstück aufweist und an dessen anderem, distalen Ende eine zum Schaft abwinkelbare, Instrumentenspitze angeordnet ist. In der Instrumentenspitze ist ein Maulteil drehbar gelagert. Des weiteren ist ein Getriebemechanismus vorgesehen, welcher zumindest eine Bewegung der Betätigungsvorrichtung in eine Rotation des Maulteils unter einem ersten bestimmten Übersetzungsverhältnis zur Betätigungsbewegung transformiert.

Angesichts dieses Stands der Technik ist es eine Aufgabe der Erfindung, ein gattungsgemäßes chirurgisches Instrument mit hoher Funktionalität zu schaffen.

Diese Aufgabe wird erfindungsgemäß durch ein chirurgisches Instrument mit den Merkmalen des Patentanspruchs 1 gelöst.

Erfindungsgemäß hat das chirurgisches Instrument u.a. einen Hohlschaft, an dessen einem, proximalen Ende eine Betätigungsvorrichtung und an dessen anderem, distalen Ende eine zum Schaft abwinkelbare Instrumentenspitze angeordnet ist, welches ein Maulteil trägt. Erfindungsgemäß ist ein Getriebemechanismus vorgesehen, welcher zumindest eine erste Bewegung der Betätigungsvorrichtung, vorzugsweise auslösbar durch die Handrotation einer Bedienerperson, in eine Rotation des Maulteils unter einem ersten bestimmten Übersetzungsverhältnis zur ersten Betätigungsbewegung transformiert. Hierdurch ist es möglich, die Instrumentenspitze, insbesondere das Maulteil trotz der relativ eingeschränkten Bewegungsmöglichkeit einer menschlichen Hand um beispielsweise bis zu 300° zu rotieren (wobei natürlich auch größere oder kleinere Rotationsgrädzahlen je nach Einsatzzweck durch entsprechende Wahl des Übersetzungsverhältnisses möglich sind) und damit komplizierte Bewegungsabläufe ohne "Umgreifen" zu realisieren. Vorteilhaft ist es hierbei, wenn der Kraft- oder Momentübertragungsmechanismus die Betätigungsvorrichtung quasi unmittelbar mit der Instrumentenspitze wirkverbindet, d.h. auf eine teure und komplizierte Sensorik z.B. zur elektronischen Erfassung von Betätigungsbewegungen und Berechnung und Übertragung von Bewegungssignalen auf die Instrumentenspitze bzw. deren Antrieb verzichtet werden kann. Diese bevorzugte Unmittelbarkeit des erfindungsgemäßen Instruments verschafft einem Operateur einen leichten Einstieg in die minimal invasive Chirurgie bzw. beschleunigt den Lernprozess im Umgang mit dem erfindungsgemäßen Instrument.

Eine vorteilhafte Ausgestaltung der Erfindung sieht ferner vor, dass die abwinkelbare Instrumentenspitze eine hülsen- oder gehäuseförmige Aufnahme zur rotierbaren Lagerung des Maulteils bildet, welche selbst jedoch rotationsfest aber schwenkbar am distalen Ende des Hohlschafts gelagert ist. Der Getriebemechanismus transformiert dabei eine zweite Bewegung der Betätigungsvorrichtung, vorzugsweise auslösbar durch ein Handabwinkeln der Bedienerperson, in ein Verschwenken bzw. Abwinkeln der Aufnahme bezüglich des Hohlschafts unter einem zweiten bestimmten Übersetzungsverhältnis zur entsprechenden zweiten Betätigungsbewegung und zwar völlig entkoppelt von der ersten Betätigungsbewegung.

Weitere vorteilhafte Ausgestaltungen der Erfindung sind Gegenstand der übrigen Unteransprüche.

Die Erfindung wird nachstehend anhand bevorzugter Ausführungsbeispiele unter Bezugnahme auf die begleitenden Zeichnungen näher erläutert.

Es zeigen:
Fig. 1 die Seitenansicht eines chirurgischen Instruments für minimal invasive Chirurgie gemäß einem ersten bevorzugten Ausführungsbeispiel der Erfindung,

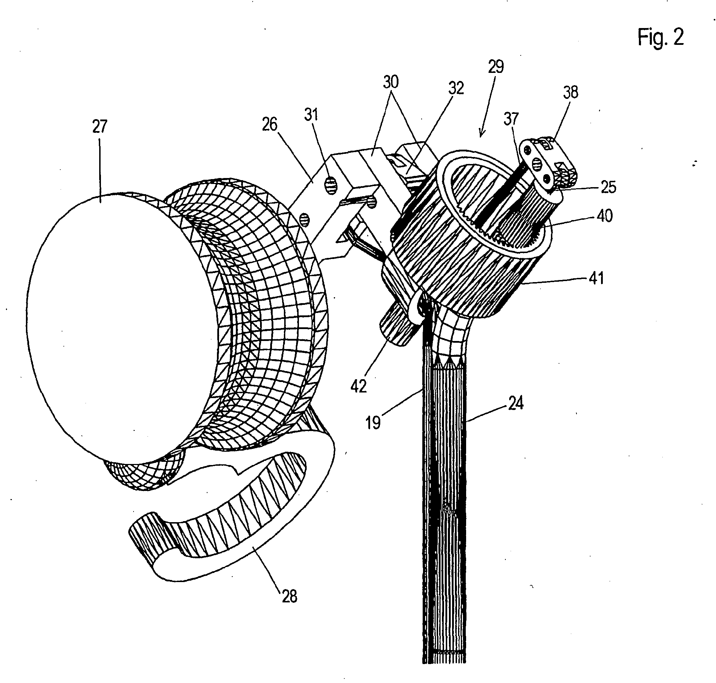

Fig. 2 die aufgeschnittene Perspektivenansicht einer Betätigungsvorrichtung gemäß dem ersten bevorzugten Ausführungsbeispiel,
Fig. 3 die teilweise aufgebrochene Seitenansicht der Betätigungsvorrichtung gemäß Fig. 2,
Fig. 4 die Draufsicht der Betätigungsvorrichtung gemäß Fig. 2,
Fig. 5 die Seitenansicht eines abwinkelbaren Bauteils mit darin gelagertem Maulteil,
Fig. 6 die Seitenschnittansicht eines abwinkelbaren Bauteils mit drehbar gelagertem Maulteil gemäß dem zweiten Ausführungsbeispiel,

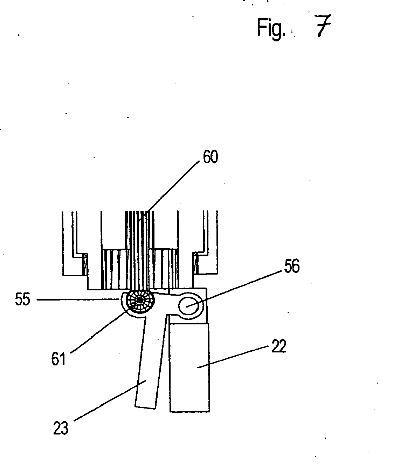

Fig. 7 die Seitenansicht des Maulteils gemäß Fig. 6,
Fig. 8 die teilweise aufgebrochene Schnittansicht des abwinkelbaren Bauteils gemäß Fig. 6,
Fig. 9 die Detailansicht eines Umlenkgestänges für die Rotation und die Betätigung des Maulteils in ihrer relativen Position zueinander,
Fig. 10 die schematisierte Seitenansicht eines Anschlussabschnitts einer Instrumentenspitze an einen Hohlschaft gemäß einem zweiten bevorzugten Ausführungsbeispiel der Erfindung,
Fig. 11, 11a die perspektivische Querschnittsansicht einer Instrumentenspitze mit hydrauLischen oder pneumatischem Antrieb bzw. Getriebe gemäß einem dritten bevorzugten Ausführungsbeispiel der Erfindung,
Fig. 12 die schematisierte Seitenansicht eines Anschlussabschnitts einer Instrumentenspitze an einen Hohlschaft gemäß einem vierten bevorzugten Ausführungsbeispiel der Erfindung,
Fig. 13 die Draufsicht des Anschlussabschnitts gemäß Fig. 12 und
Fig. 14 und 15 die schematisierten Seitenansichten eines Anschlussabschnitts einer Instrumentenspitze an einem Hohlschft gemäß einem fünften bevorzugten Ausführungsbeispiel der Erfindung.

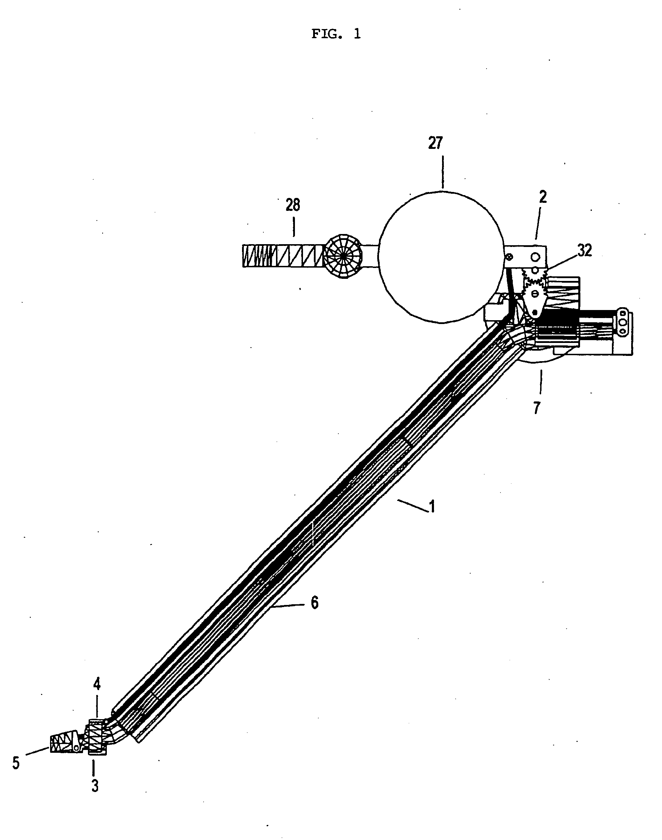

Wie aus der Fig. 1 zu entnehmen ist, besteht das chirurgische Instrument gemäß dem ersten bevorzugten Ausführungsbeispiel der Erfindung im wesentlichen aus den folgenden Baugruppen:
- einem Hohlschaft 1 vorzugsweise aus einem biege- und torsionssteifen Material oder Aufbau,
- einer Betätigungsvorrichtung 2, welche an einem axialen Endabschnitt des Hohlschafts 1 vorgesehen ist,
- einer Instrumentenspitze 3 bestehend aus einer gehäuseartigen Aufnahme 4 zur drehbaren Lagerung eines Maulteils 5, die an dem anderen axialen Endabschnitt des Hohlschafts 1 verschwenkbar angelenkt ist und
- einem Kraft- und/oder Drehmomentübertragungsmechanismus (Getriebemechanismus), über den sowohl die Aufnahme 4 wie auch das Maulteil 5 mit der Betätigungsvorrichtung 2 mechanisch zu deren vorzugsweise unabhängige Betätigungen gekoppelt sind.

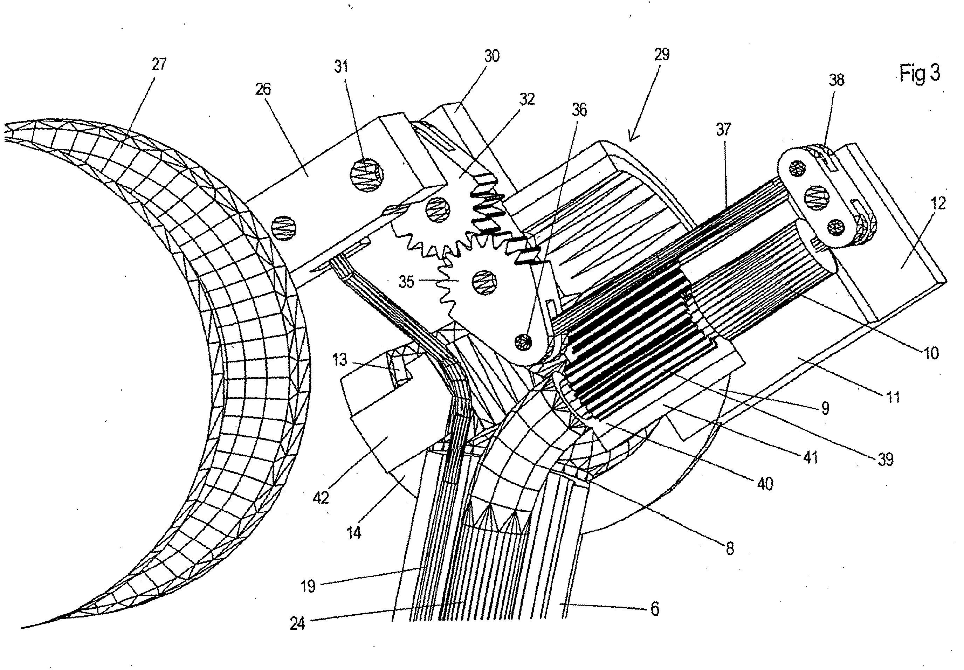

Der Hohlschaft 1 wird gebildet durch ein längliches, im wesentlichen biegesteifes Rohrstück 6 aus einem nicht oxidierenden Metall, welches an seinen axialen Enden rechtwinklig auf ein vorbestimmtes Maß abgeschnitten ist. An dem einen, nachfolgend als proximaler Endabschnitt bezeichneten axialen Ende des Rohrstücks 6 ist gemäß der Fig. 3 eine Kugelkalotte 7 beispielweise durch Hartlöten, Schweißen oder Schrauben angesetzt, die eine Eintrittsbohrung 8 in etwa vom Durchmesser entsprechend des Rohrstück-Innendurchmessers hat, welche axial fluchtend in das Rohrstück 6 mündet und eine Austrittbohrung 9 von im wesentlichen gleichem Durchmesser hat, deren Mittelachse in einem Winkel von zwischen 100° und 150°, vorzugsweise 135° zur Eintrittsbohrungs-Mittelachse ausgerichtet ist.

An die Austrittsbohrung 9 schließt sich ein mit einer Längsbohrung 10 versehener Fortsatz oder Sockel 11 an, der vorzugsweise einstückig mit der Kugelkalotte 7 an deren Außenseite ausgebildet ist, derart, dass dessen Längsbohrung 10 in einem bestimmten Parallelabstand zur Mittelachse der Austrittsbohrung 9 in diese mündet. Diese Längsbohrung 10 hat dabei einen kleineren Durchmesser als jener der Austrittsbohrung 9. An dem freien Endabschnitt des Fortsatzes 11 ist zudem eine Anlenkstelle 12 zur schwenkbaren Anlenkung von Hebeln und/oder Stangen des Kraftübertragungsmechanismus ausgebildet, wie nachfolgend noch beschrieben wird.

An einem durch die Eintritts- und Austrittsbohrung 8, 9ausschließenden Kugelabschnitt ist ein Kugelsegment von der Kugelkalotte 7 abgeschnitten, wodurch ein freier Zugang 13 in das Innere der Kugelkalotte 7 geschaffen wird. In dem verbleibenden Kugelsegment zwischen der Eintrittsöffnung 8 und dem freien Zugang 13 ist dabei eine Lagerbohrung 14 in der Kugelkalotte 7 ausgebildet, die axial fluchtend zur Ausgangsbohrung 9 ausgerichtet ist, jedoch einen zur Ausgangsbohrung 9 kleineren Durchmesser etwa entsprechend dem der Längsbohrung 10 aufweist.

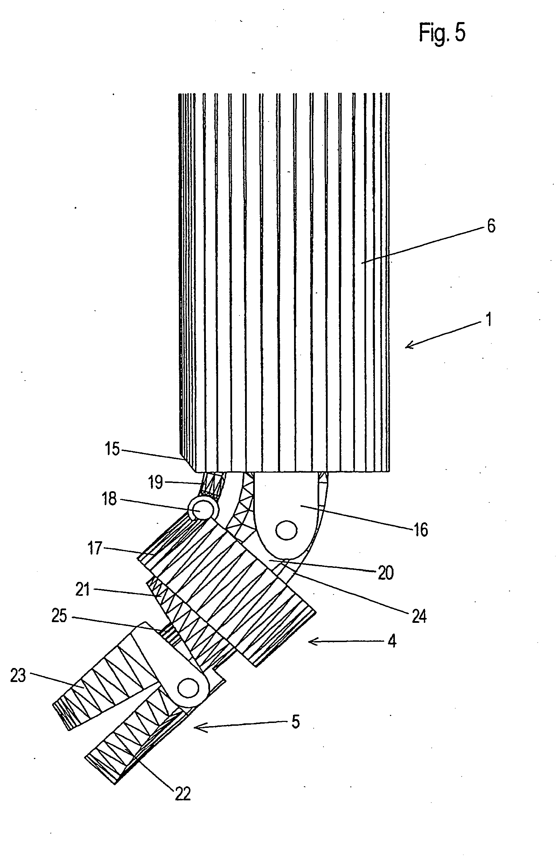

Das andere, nachfolgend als distaler Endabschnitt bezeichnete Ende des Rohrstücks 6 weist an einer Seite (gemäß der Fig. 5 die linke Seite), welche auf Seiten der Lagerbohrung 14 liegt, eine im wesentlichen gerade verlaufende Anfasung 15 im Winkel von ca. 45° zur distalen Stirnseite des Rohrstücks 6 auf. Am distalen Endabschnitt des Rohstücks 6 sind ferner zwei axial sich erstreckende Anlenklaschen 16 vorgesehen, die zu beiden Seiten der Anfasung 15 jeweils um ca. 90° zu dieser versetzt sind und an ihren freien Enden Anlenkbohrungen oder Zapfen aufweisen, deren Mittelachsen fluchtend zueinander ausgerichtet sind.

Die gehäuseartige Aufnahme 4 wird gemäß dem ersten bevorzugten Ausführungsbeispiel der Erfindung durch eine zylindrische, ringförmige Hülse 17 oder Lagerbüchse vorzugsweise mit einem Außen- und Innendurchmesser entsprechend dem Rohrstück 6 gebildet, die an ihrer einen, dem distalen Ende des Rohrstücks 6 zugewandten Stirnkante eine Anlenkstelle 18 beispielsweise in Form eines Lagerauges oder einer Gelenkkalotte für das An- bzw. Einsetzen einer Gelenkkugel hat. Die Anlenkstelle 18 ist dabei fest, vorzugsweise einstückig mit der Hülse 17 verbunden. An dieser Anlenkstelle 18 ist dreh- und/oder schwenkbar eine Aufnahme-Betätigungsstange 19 über ein entsprechend der Anlenkstelle 18 geformtes Gegenstück als Bestandteil der Betätigungsstange 19 angelenkt.

Ca. 90° zu beiden Seiten der Anlenkstelle 18 versetzt sind zwei Anlenklaschen 20 an der Hülse 17 vorzugsweise einstückig mit dieser angeordnet, die an ihren freien Enden jeweils mit einem auf einer gemeinsamen Achse liegenden Durchgangsloch oder einem Lagerzapfen versehen sind. Diese hülsenseitigen Laschen 20 sind mit den rohrstückseitigen Anlenklaschen 16 über die Zapfen und Durchgangslöcher scharnierartig verbunden, derart, dass die Hülse 17 am distalen Endabschnitt des Rohrstücks 6 verschwenkt bzw. abgewinkelt werden kann, wobei der maximale Schwenkwinkel zumindest in die eine Schwenkrichtung aufgrund der Anfasung 15 am distalen Ende des Rohrstücks 6 soweit vergrößert ist, dass die Hülse 17 beispielsweise aus einer zum Rohrstück 6 coaxialen Position um ca. 180° verschwenkt werden kann und sich in dieser verschwenkten Position im wesentlich parallel neben dem distalen Endabschnitt des Rohrstücks 6 ausrichtet.

Innerhalb der Hülse 17 lagert drehbar, jedoch schiebefest ein kolbenförmiges, eine axiale Durchgangsbohrung aufweisendes Rotationsbauteil 21, welches an seinem distalen, d.h. dem Rohrstück 6 abgewandten axialen Ende einen schnabelförmigen Vorsprung 22 hat, der entweder einstückig oder durch Schweißen oder Löten mit dem Rotationsbauteil 21 verbunden ist und sich in axialer Richtung erstreckt. Dieser Vorsprung 22 bildet eine erste, nicht individuell bewegbare Klemmbacke des Maulteils 5. Nahe dem Befestigungsbereich des Vorsprungs 22 ist scharnierförmig eine zweite Klemmbacke 23 an der ersten Klemmbacke oder dem Rotationsbauteil 21 angelenkt, der unter Ausbildung des Maulteils 5 mit der ersten Klemmbacke 22 zusammenwirkt.

An dem dem Rohrstück 6 zugewandten Ende des Rotationsbauteils 21 ist ein distales Ende einer biegsamen, jedoch torsionssteifen Antriebswelle 24 mittig, d.h. zwischen den hülsenseitigen Anlenklaschen 20 verlaufend, befestigt, welche in dem Rohrstück 6 drehbar gelagert ist. Die längs des Rohrstücks 6 sich erstreckende biegsame Antriebswelle 24 ist mit einem axialen Durchgangsloch versehen, das mit der Durchgangsbohrung im Rotationsbauteil 21 im wesentlichen fluchtet. An dieser Stelle sei darauf hingewiesen, dass das Rotationsbauteil 21 auch einstückig mit der biegsamen Antriebswelle 24 ausgebildet sein kann.

Innerhalb des Durchgangslochs lagert relativ zur Antriebswelle 24 bewegbar ein Betätigungszug 25 für die zweite, scharnierartig bewegbare Klemmbacke 23, der durch die Durchgangsbohrung im Rotationsbauteil 21 hindurchgeführt und an der zweiten Klemmbacke 23 für deren Verschwenken bei Zug- und/oder Druckbelastung montiert ist.

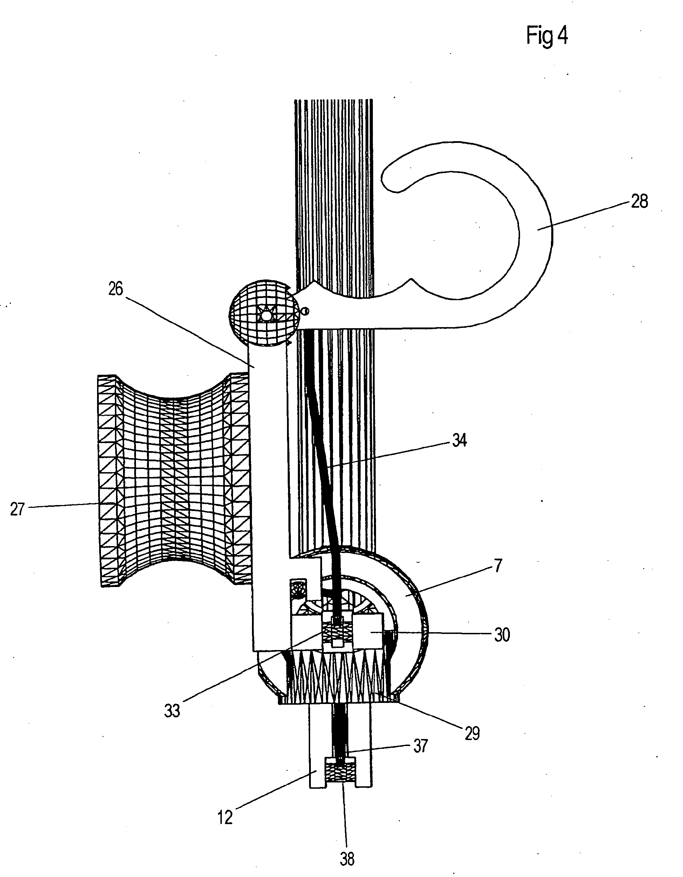

Die Betatigungsvorrichtung 2 ist an und in der am proximalen Endabschnitt des Hohlschafts 1 befestigten Kugelkalotte 7 montiert. Sie hat gemäß der Fig. 3 und 4 eine Handhabe in Form eines Hebels 26, an dem vorzugsweise eine Art Knopf 27 oder ein andersartig gestaltetes Griffstück befestigt ist, welches von einer Bedienerperson vorzugsweise mit Daumen und Zeigefinger einer Hand umgriffen und festgehalten werden kann. Des weiteren ist an dem Hebel 26 an einer bezüglich des Knopfs 27 ergonomisch geeigneten Position eine relativ zu dem Hebel 26, verschwenkbare Klinke 28 oder Abzug als weiterer Bestandteil der Handhabe angeordnet, die so gestaltet ist, dass sie mit zumindest einem der noch verbleibenden Finger der einen Hand betätigbar ist.

Der Hebel 26 selbst ist an seinem zur Klinke 28 entfernten, hinteren Endabschnitt an einem Lagerkopf 29 anscharniert, der wiederum drehbar in der Kugelkalotte 7 gelagert ist. Hierfür bildet der Lagerkopf 29 eine in der Ansicht gemäß der Fig. 4 im wesentlichen U-förmige Anlenkstütze 30, an deren freiem Endabschnitt ein seitlicher Schwenkzapfen (nicht weiter gezeigt) angeordnet ist. Der Hebel 26 hat dementsprechend an seinem hinteren Endabschnitt eine Querbohrung 31, in welche der Schwenkzapfen eingesteckt ist. Zwischen den Schenkeln der U-förmigen Anlenkstütze 30 ist darüber hinaus ein erstes Zahnrad oder Ritzel-32 drehbar an einem nicht gezeigten Bolzen oder Stift gehalten, der im Höhenabstand (unterhalb) zum Schwenkzapfen an der Anlenkstütze 30 ausgebildet ist. Dieses erste Ritzel 32 vorzugsweise in Form eines Nockens hat exzentrisch einen Anlenkpunkt 33 in der Konstruktionslage gemäß der Fig. 3 etwa auf Höhe des Schwenkzapfens für das Befestigen einer Zug- /Druckstange 34. Die Zug-/Druckstange 34 erstreckt sich im wesentlichen längs des Hebels 26 und ist an ihrem anderen, freien Ende mit der Klinke 28 verbunden.

Mit dem ersten Ritzel 32 in Kämmeingriff befindend ist ein zweites, ebenfalls vorzugsweise nockenförmig gestaltetes Ritzel 35, welches schwenkbar am Lagerkopf 29 gehalten ist und einen exzentrischen Anlenkpunkt 36 hat, an dem eine Verlängerungsstange 37 montiert ist. Die Verlängerungsstange 37 erstreckt sich dabei längs des Fortsatzes 11 bis zu dessen äußerer Anlenkstelle 12. An dieser Anlenkstelle 12 ist ein Kipphebel 38 mittig gelagert, an dessen einem äußeren Ende die Verlängerungsstange 37 angelenkt ist. An dem anderen äußeren Ende des Kipphebels 38 ist ferner das proximale Ende des Betätigungszugs 25 vorzugsweise über einen Kugelkopf für eine Schwenk- und Torsionsbewegung angelenkt.

Die Klinke 28, die Zug-Druckstange 34, die beiden in Kämmeingriff stehenden Nockenritzel 32; 35, die Verlängerungsstange 37, der Kipphebel 38 sowie der Betätigungszug 25 in dieser Reihenfolge bilden eine Maulteilbetätigung als ein Getriebezug des Kraft- und/oder Drehmomentübertragungsmechanismus.

An dem Hebel 26 ist zwischen seiner Lagerung an der Anlenkstütze 30 und der Klinke 28 die Aufnahme-Betätigungsstange 19 angelenkt. Diese verläuft ebenfalls durch das Rohrstück 6 parallel zur biegsamen torsionssteifen Welle 24 und tritt an dem Zugang 13 in das Kugelkalotten-Innere aus dem Hohlschaft 1 aus. In diesem Bereich hat die Betätigungsstange 19 zumindest ein kardanisches Gelenk oder einen schubstarren biegsamen Stangenabschnitt (nicht näher dargestellt), an welchem die Betätigungsstange 19 zum Anlenkpunkt an dem Hebel 26 abgekrümmt ist.

Die biegsame, torsionssteife Welle oder Schlauch 24 ist nach deren Austritt aus dem Rohrstück 6 innerhalb der Kalotte 7 in Richtung zur Längsbohrung 10 des Fortsatzes 11 gebogen und in der Längsbohrung 10 drehbar gelagert. Im Bereich jenes Wellenabschnitts, der sich innerhalb der Kugelkalotte 7 erstreckt, ist die biegsame Welle 24 mit einer Außenverzahnung 39 versehen. Mit der Außenverzahnung 39 ist eine Innenverzahnung 40 in Kämmeingriff, welche an dem Lagerkopf 29 ausgebildet ist. Hierfür weist der Lagerkopf 29 eine Art Hülse 41 auf, die mit vorstehend genannten Innenverzahnung 40 versehen ist und die biegsame Welle 24 umgreifend in der Kugelkalotte 7 schwenkbar gelagert ist. Zur besseren Führung des Lagerkopfs 29 weist dieser zusätzlich einen Schwenkzapfen 42 auf, der sich co-axial zur Hülse 41 bzw. deren Mittelachse erstreckt und in der Lagerbohrung 14 mit kleinem Durchmesser in der Kugelkalotte 7 im Bereich zwischen der Eintrittbohrung 8 und dem Kalottenzugang 13 gelagert ist.

Zur Funktionsweise des chirurgischen Instruments gemäß dem ersten bevorzugten Ausführungsbeispiel wird folgendes ausgeführt:

Wie vorstehend bereits kurz angedeutet wurde, soll bei chirurgischen Instrumenten dieser Gattung sowohl das Maulteil per se betätigbar sein, d.h. die Klemmbacken geöffnet und geschlossen werden können, als auch eine Verschwenkung bzw. Abwinklung des Maulteils d.h. der Instrumentenspitze um mindestens 180° bezüglich des Rohrstücks 6 ermöglicht werden. Darüber hinaus soll das Maulteil eine zumindest 360°-Rotation um die Längsachse der Instrumentenspitze d.h. der abwinkelbaren Aufnahme 4 ausführen können.

Das Öffnen und Schließen des Maulteils 5 erfolgt über die Klinke 28 als individuelles Betätigungselement, welche mit einem oder mehreren Fingern einer Hand einer Bedienerperson betätigt wird. Hierfür ergreift die Bedienerperson das erfindungsgemäße Instrument mit Daumen und Zeigefinger am Knopf 27 und legt die restlichen drei Finger an der Klinke 28 an. Durch Ziehen der Klinke 28 in Richtung Knopf 27 wird eine Druckkraft auf die Verbindungsstange (oder auch Excenterstange) 34 zwischen der Klinke 28 und dem ersten Ritzel 32 aufgebracht, wodurch das erste Ritzel 32 gedreht wird und dabei das zweite Ritzel 35 antreibt. Hierdurch wird die exzentrisch am Ritzel 35 angelenkte Verlängerungsstange 37 mit einer Kraft beaufschlagt und verschwenkt den daran angeschlossenen Kipphebel 38. Diese Verschwenkbewegung wird in eine Translationsbewegung des Maulteil-Betätigungszugs 25 umgewandelt, der am anderen Ende des Kipphebels 38 angelenkt und innerhalb der biegsamen Welle 24 entsprechend beispielsweise einer Fernbetätigung für den Auslöser an einem Fotoapparat verschiebbar geführt ist. Durch die Translationsbewegung des Betätigungszugs 25 wird die daran angelenkte zweite Klemmbacke 23 in Schließrichtung verschwenkt. Durch Drücken der Klinke 28 in entgegengesetzte Richtung erfolgt eine Verschwenkung der zweiten Klemmbacke 23 in Öffnungsrichtung.

Wird nunmehr der Hebel 28 um dessen hinteren Anlenkpunkt 31 entsprechend einer beispielsweise aufwärts gerichteten Handbewegung im Bereich des Handgelenks aus der Konstruktionslage gemäß der Fig. 3 nach oben verschwenkt, wird hierdurch die am Hebel 28 zwischen Anlenkpunkt 31 und Knopf 27 befestigte Aufnahme-Betätigungsstange 19 aus der Zugangsöffnung 13 der Kugelkalotte 7 gezogen, wobei demzufolge eine Zugkraft auf die am distalen Ende des Rohrstücks 6 angelenkte Hülse 17 über die Anlenkstelle 18 der Betätigungsstange 19 übertragen wird. Dementsprechend wird die Hülse 17 um die Schwenkzapfen an den Rohrstück-seitigen Laschen 16 verschwenkt und folgt somit der schwenkenden Handbewegung der Bedienerperson. Dabei erfährt die Welle 24 in dem Abschnitt zwischen dem distalen Ende des Rohrstücks 6 und der Hülse 17 eine entsprechende Abwinklung.

Wird nunmehr die Hand der Bedienerperson um die Längsachse des Unterarms gedreht, wird hierdurch der Lagerkopf 29 um dessen Schwenkzapfen 42 und dessen Hülse 41 innerhalb der Kugelkalotte 7geschwenkt. Durch diese Schwenkbewegung wird die biegsame Welle 24 über die Innen- Außenverzahnung 39; 40 mit einem bestimmten Übersetzungsverhältnis um ihre Längsachse gedreht. D.h. die Welle 24 dreht innerhalb der Längsbohrung 10 des Fortsatzes 11 und innerhalb des Rohrstücks 6, wobei die Winkelposition des Fortsatzes 11 relativ zum Rohrstück 6 zu einer fortlaufenden Verbiegung der Welle 24 innerhalb der Kugelkalotte führt. Durch die Rotation der biegsamen Welle 24 wird das Rotationsbauteil 21 und damit das daran montierte Maulteil 5 innerhalb der Aufnahme 4 gedreht, während die Aufnahme 4 selbst durch die scharnierartige Anlenkung an das Rohrstück 6 festgehalten wird.

Bei der Rotation der Welle 24 im Rohrstück 6 wird der Maulteil-Betätigungszug 25 reibungsbedingt ebenfalls mitgedreht. Die Kugelkopfanlenkung des Betätigungszugs 25 an den Kipphebel 38 lässt indessen eine solche Rotation zu, ohne dass der Betätigungszug 25 Torsionskräften ausgesetzt wird. Bei der Verschwenkung des Hebels 26 um seinen hinteren Anlenkpunkt 31 verändert sich die auch die Position der Klinke 28 bezüglich des ersten Ritzels 32. Da die Anlenkung der Verbindungsstange 37 zwischen dem Ritzel 32 und der Klinke 28 jedoch im wesentlichen auf Höhe des Anlenkpunkts 31 des Hebels 26 erfolgt, bleibt das erste Ritzel 32 beim Verschwenken des Hebels 26 im wesentlichen unbetätigt. Das Kreuzgelenk bzw. der biegsame Abschnitt der Betätigungsstange 19 zur Verschwenkung der Aufnahme 4 befindet sich in etwa auf der Schwenkachse des Lagerkopfs 29 innerhalb der Kugelkalotte 7. Dadurch bleibt die Längsposition der Aufnahme-Betätigungsstange 19 von einer Schwenkbewegung des Lagerkopfs 29 unbeeinflusst.

Das Übersetzungsverhältnis zur Betätigung des Maulteils 5, d.h. der zweiten Klemmbacke 23 lässt sich an den Ritzeln 32; 35, der Anlenkstelle der Verbindungsstange 34 an der Klinke 28 und/oder dem Hebelverhältnis am Kipphebel 38 einstellen. Das Übersetzungsverhältnis zur Betätigung der Aufnahme 4 ist am geeignetsten durch die Wahl der Anlenkung der Aufnahme-Betätigungsstange 19 am Hebel 26 einstellbar. Das Übersetzungsverhältnis zur Rotation des Maulteils 5 wird schließlich durch die Wahl der Innen- und Außenverzahnung 39; 40 an der Hülse 41 des Lagerkopfes 29 bzw. an der biegsamen Welle 24 bestimmt. Vorzugsweise wird bei einer Rotationsbewegung des Hebels 26 und damit des Lagerkopfs 29 von 90° eine Maulteilrotation von bis zu 300° erzielt, wobei die Drehachse für die Rotation des Handgelenks entsprechend der Anordnung der Eintritts- und Austrittsbohrung 8, 9 um ca. 45° zur Hohlschaftachse geneigt ist.

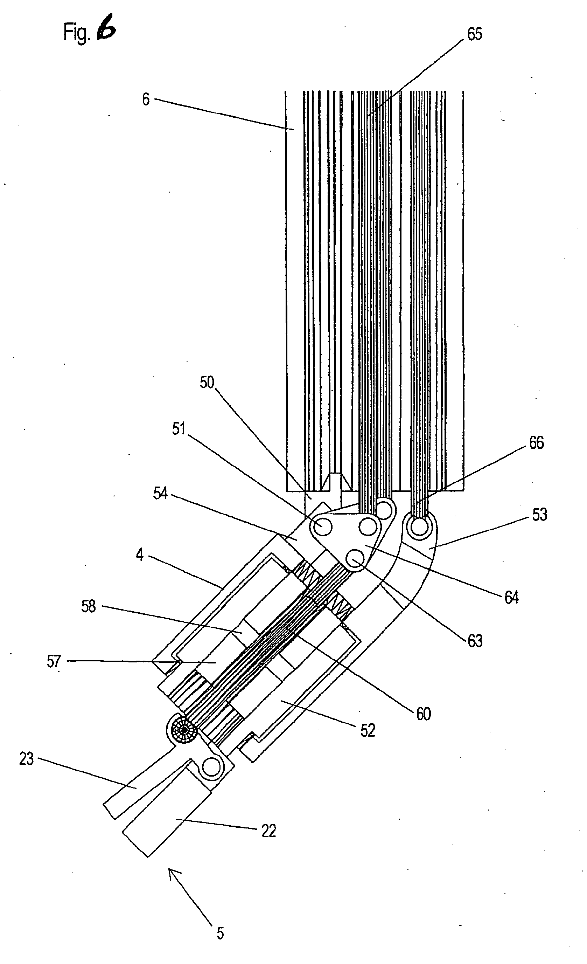

Gemäß der Fig. 6 hat die Instrumentenspitze 3 eine gehäuseartige, im wesentlichen zylindrische Aufnahme 4, in der ein zylindrischer Rotationskörper 52 drehbar gelagert, jedoch axial festgelegt ist. An einer zum Rohrstück 6 zugewandten Stirnseite ist die Aufnahme 4 mit einem axial sich erstreckenden exzentrisch, vorzugsweise randseitig angeordneten Bügel 53 ausgebildet, an dessen freiem Ende eine Anlenkstelle in Form eines Durchgangslochs oder einer Kugelkopfschale ausgebildet ist. An der zum Bügel 53 gegenüberliegenden Randseite der Aufnahme 4 ist eine axiale Lasche 54 angeordnet, die an ihrem freien Ende ebenfalls mit einer Durchgangsbohrung ausgebildet und mittels eines Stifts 51 an den axialen Vorsprung 50 des Rohrstücks 6 schwenkbar anscharniert ist.

An dem distalen Ende des Rotationskörpers 52 ist ein axial sich erstreckender Vorsprung 22 vorzugsweise einstückig ausgebildet, der eine erste Klemmbacke eines Maulteils 5 bildet. An diesem Vorsprung 22 ist ferner eine zweite Klemmbacke 23 am Punkt 56 anscharniert, die an einem in etwa in der Mittelachse des Rotationskörpers 52 liegenden Backenabschnitt eine Anlenkstelle 55 in Form einer Kugelkopfschale hat. Innerhalb des Rotationskörpers 52 ist ein axial verschiebbarer drehfester Kolben 57 gelagert, der an seiner Mantelfläche eine Spiralnut (nicht näher gezeigt) aufweist. Der Rotationsköper 52 hat dabei zumindest einen radial nach innen ragenden Mitnahmestift 58, der in die Spiralnut gleitend eingreift, derart, das bei einer axialen Verschiebung des Kolbens 57 der Rotationskörper 52 durch das Zusammenwirken der Spiralnut und des Mitnahmestifts 58um seine Längsachse gedreht wird.

Der Kolben 57 ist ferner mit einer zentralen Durchgangsbohrung 59 versehen, die sich axial erstreckt und in etwa fluchtend zur Anlenkstelle 55 der zweiten beweglichen Klemmbacke 23 des Maulteils 5 ausgerichtet ist. Innerhalb dieser Durchgangsbohrung 59 ist ein Zug-/Druckstab 60 axial verschiebbar gelagert, an dessen distalem Ende ein Kugelkopf 61 ausgebildet ist, der in der Anlenkstelle 55 der zweiten Klemmbacke 23 aufgenommen ist. Der Zug-/Druckstab 60 weist dabei in einem Abschnitt ein Gelenk oder eine biegsames Zwischenstück 62 auf. An seinem proximalen Ende ist der Zug-/Druckstab 60 ferner mit einem Kugelkopf oder einem Lagerauge 63 versehen.

Wie insbesondere aus der Fig. 6 zu entnehmen ist, umfasst der Kraft-/Drehmomentübertragungsmechanismus im Bereich der Instrumentenspitze eine zentrale Umlenkplatte 64, die an der Anlenkstelle der Lasche 54, bzw. dem Scharnierstift 51 schwenkbar angelenkt ist, welcher durch die Durchgangsbohrung der Lasche 54 sowie des rohrstückseitigen Vorsprungs 50 gesteckt ist. Die zentrale Umlenkplatte 64 ist vorliegend von dreiecksförmiger Gestalt und weist zwei weitere serielle (in den Eckbereichen der Platte angeordnete) Durchgangslöcher auf. An einem dieser Durchgangslöcher ist die Zug-/Druckstange 60 anscharniert, während an dem anderen Durchgangsloch eine Maulteilbetätigungsstange 65 anscharniert ist, die im Rohrstück 6 geführt ist.

Des weiteren hat der Kraft-/Drehmomentübertragungsmechanismus eine Aufnahmebetätigungsstange 66, die ebenfalls im Rohrstück 6 geführt und an der Anlenkstelle des Bügels 53 anscharniert ist.

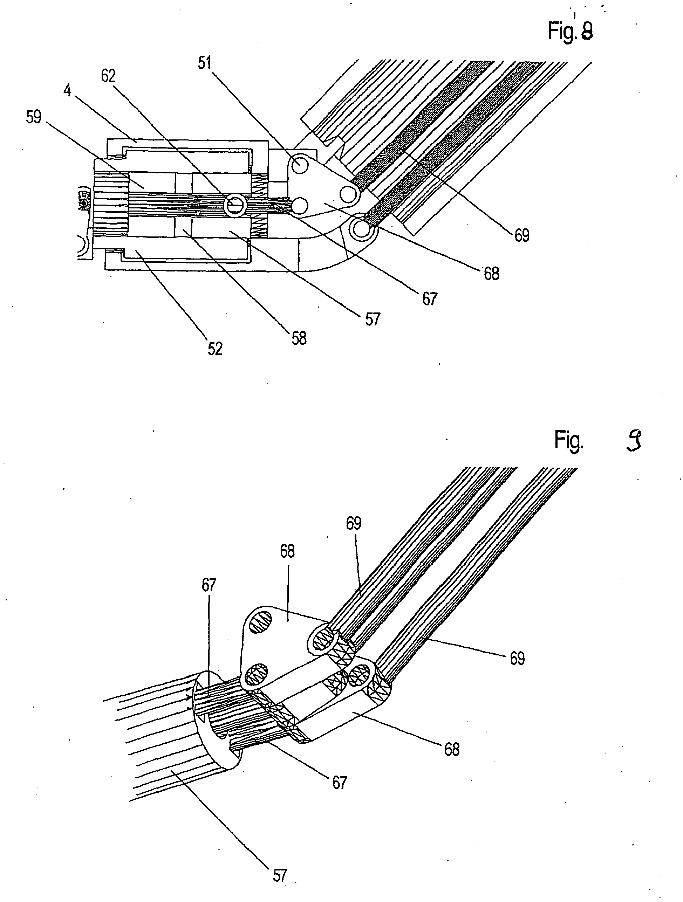

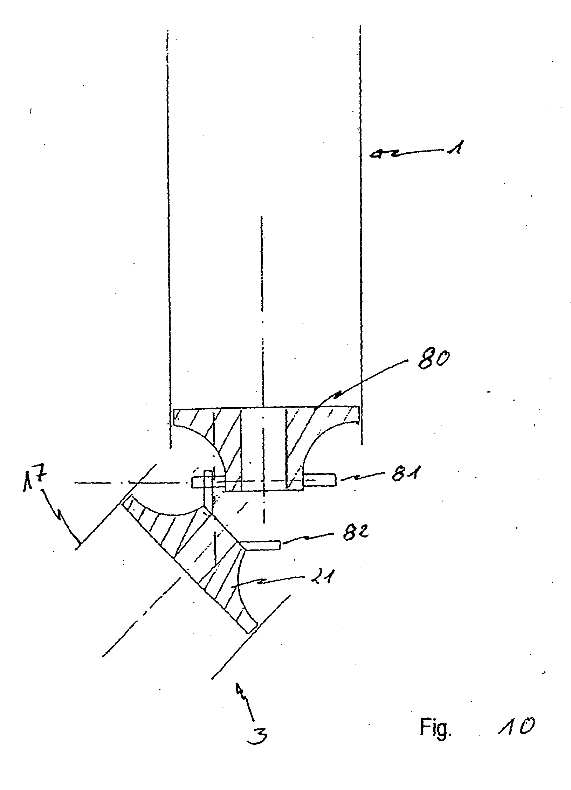

Wie aus den Fig. 8 und 9 zu entnehmen ist, sind an dem Kolben 57 beidseits der Zug-/Druckstange 60 zwei axiale Bolzen 67 fixiert, die an ihren an dem Kolben 57 vorragenden Enden jeweils ein Lagerauge aufweisen. An dem Scharnierstift 51 zur schwenkbaren Lagerung der Aufnahme 4 am Rohrstück 6 sind zwei weitere seitliche Umlenkplatten 68 beidseits der zentralen Umlenkplatte 64 mit hierzu identischer Konstruktion schwenkbar angeordnet, an denen die beiden Bolzen 67 jeweils angelenkt sind. An den seitlichen Umlenkplatten 68 sind ferner jeweils eine Kolbenbetätigungsstange 69 anscharniert, welche ebenfalls in dem Rohrstück 6 geführt sind.

Sämtliche Betätigungsstangen sind an der Betätigungsvorrichtung 2 angelenkt.

Nachfolgend werden zu den vorstehenden Beispielen alternative Getriebe- bzw. Kraft- und Momentübertragungsmechanismen anhand weiterer Ausführungsbeispiele der Erfindung beschrieben, welche sämtlich die Unmittelbarkeit bei der Übertragung einer Betätigungsbewegung der Betätigungsvorrichtung auf die Instrumentenspitze vorzugsweise unter einem vorbestimmten Übersetzungsverhältnis gemeinsam haben.

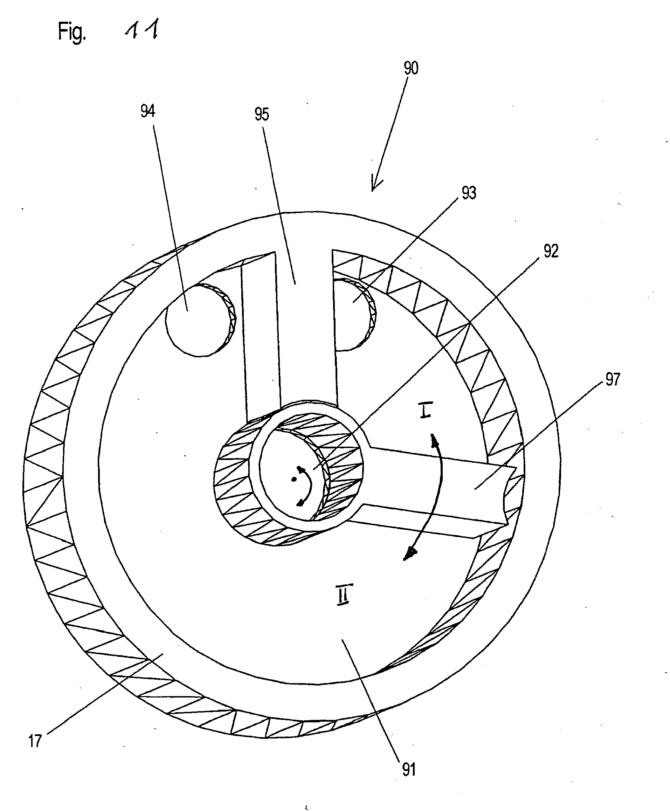

Die Fig. 10 zeigt demzufolge ein zweites Ausführungsbeispiel der Erfindung im Scharnierbereich der Instrumentenspitze 3 und des Hohlschafts 1. Sämtliche weitere technische Ausgestaltungen des zweiten Ausführungsbeispiels, insbesondere der Betätigungsvorrichtung sowie der Lagerung des Maulteils können dem ersten Ausführungsbeispiel entsprechen und brauchen daher an dieser Stelle nicht mehr näher erläutert zu werden.

Gemäß der Fig. 10 ist die im ersten Ausführungsbeispiel verwendete torsionssteife, biegsame Welle zumindest teilweise durch eine starre Drehwelle 80 ersetzt. D.h. zumindest im Anschlussbereich von Instrumentenspitze 3 und Hohlschaft 1 ist die aus dem ersten Ausführungsbeispiel bekannte biegsame-Welle (in Fig. 10 nicht mehr gezeigt) axial an die Starrwelle 80 angeschlossen, die an ihrem distalen Ende am distalen Endabschnitt des Hohlschafts mit einer Außenverzahnung beispielsweise einem kugelig gewölbten Kegelzahnrad oder einer Anzahl von Radialstiften 81 versehen ist. Das ebenfalls aus dem ersten Ausführungsbeispiel bekannte Rotationsbauteil 21 ist an seinem zum Hohlschaft 1 gewandten Endabschnitt mit einer entsprechend gestalteten Außenverzahnung beispielsweise in Form von ca. 45° zu deren Längsachse radial nach außen stehenden Stiften 82 versehen, die mit den Radialstiften 81 der Starrwelle 80 in Eingriff sind, um ein Drehmoment von der Starrwelle 80 auf das Rotationsbauteil 21 zu übertragen. Die Instrumentenspitze 3 selbst ist an den Hohlschaft 1 wie beim ersten Ausführungsbeispiel angelenkt, wobei auch der Betätigungszug für das Maulteil (beides in Fig. 12 nicht gezeigt) entsprechend innerhalb der Starrwelle 80 und innerhalb des Rotationsbauteils 21 verlegt ist.

Die Funktionsweise des Instruments gemäß dem zweiten Ausführungsbeispiel entspricht jener des ersten Ausführungsbeispiels mit der Ausnahme, dass bei einer Abwinklung der Instrumentenspitze 3 bezüglich des Hohlschafts 1 die Radialstifte 81 bezüglich der gegenüberliegenden Stifte 82 des Rotationsbauteils 21 ihre jeweiligen Eingriffswinkel verändern.

Dabei sei darauf hingewiesen, dass beim zweiten Ausführungsbeispiel auf die torsionssteife, biegsame Welle gänzlich verzichtet werden kann, also auch im Bereich der Betätigungsvorrichtung. Wird nämlich im Scharnierbereich zwischen der Instrumentenspitze 3 und dem Hohlschaft 1 das vorstehend beschrieben Getriebe (Stifte 81 und Radialstifte 82)zwischengefügt, ist die Biegsamkeit der Welle an dieser Stelle nicht mehr erforderlich. Da die Welle gemäß dem ersten Ausführungsbeispiel im Bereich der Betätigungsvorrichtung zwar ebenfalls abgewinkelt ist, wobei die Biegsamkeit der Welle vorteilhaft ausgenutzt wird, diese Abwinklung jedoch unverändert bleibt, könnte an dieser Stelle auch ein Gelenk vorgesehen sein, das zwei Einzelteile einer nunmehr vollständig starr ausgebildeten Welle miteinander verbindet.

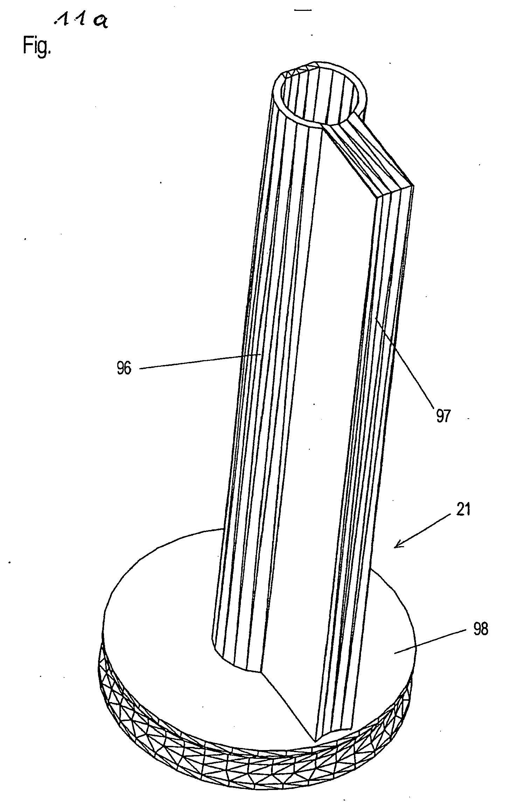

Bei dem Ausführungsbeispiel gemäß der Fig. 11 wird zusätzlich für die Kraft- und Drehmomentübertragung eine hydraulisch oder pneumatisch arbeitende Einrichtung 90 zwischen dem Rotationsbauteil und der Betätigungseinrichtung (beides in Fig. 11 nicht gezeigt) im Getriebemechanismus zwischengefügt.

Prinzipiell soll eine manuelle Betätigungsbewegung der Betätigungsvorrichtung auf die Instrumentenspitze übertragen werden, derart, dass das Maulteil innerhalb der Instrumentenspitze eine Rotation ausführt. Dies kann, wie anhand der vorstehend beschriebenen Ausführungsbeispiele ausgeführt wurde, durch einen rein mechanischen Getriebemechanismus aber auch durch einen kombinierten mechanisch-hydraulischen/pneumatischen Getriebemechanismus erfolgen. Im dritten Ausführungsbeispiel gemäß Fig. 11 und 11a ist das aus dem ersten Ausführungsbeispiel bekannte Rotationsbauteil als Rotationskolben 21 ausgebildet, der in der Instrumentenspitze, d.h. der Hülse 17 drehbar gelagert ist. Die Hülse 17 selbst ist wie im ersten Ausführungsbeispiel abwinkelbar am Hohlschaft gelagert und wird in Übereinstimmung mit dem ersten Ausführungsbeispiel verschwenkt.

Die Hülse 17 ist gemäß diesem Ausführungsbeispiel jedoch zumindest an ihrem dem Hohlschaft zugewandten Ende mit einer Stirnwand 91 verschlossen, in die drei Durchgangsbohrungen 92, 93, 94, d.h. eine Zentralbohrung 92 sowie zwei dezentral angeordnete Druckanschlussbohrungen 93, 94 eingebracht sind. Die beiden dezentralen Bohrungen 93, 94 sind durch einen längsverlaufenden, zur Zentralbohrung 92 sich erstreckenden Teilungssteg 95 getrennt, der an seiner radial inneren Kante einen Dichtungsstreifen (nicht gezeigt) hat. Der Rotationskolben 21 besteht gemäß der Fig. 11a aus einem Röhrchen 96, das in der Zentralbohrung 92 dichtend gelagert und an der radial inneren Kante des Stegs 95 fluiddicht geführt ist. Am distalen Ende des Röhrchens ist eine Kreisplatte 98 fixiert, welche die Hülse 17 an ihrem distalen Ende dicht verschließt. Der Rotationskolben 21 ist dabei drehbar jedoch axial im wesentlichen verschiebefest in der Hülse 21 gehalten.

Wie ferner aus der Fig. 11 zu entnehmen ist, hat das Röhrchen 96 eine axial sich erstreckende, radial vorstehende Leiste 97, die an ihrer radial äußeren Kante dichtend an der Innenseite der Hülse 21 streift. Durch das Zusammenwirken von dem Steg 95, dem Röhrchen 96 und der Leiste 97 werden zwei fluiddicht getrennte Druckräume I, II gebildet, die jeweils einen der Druckanschlüsse 93, 94 aufweisen und deren Volumen sich in Abhängigkeit voneinander je nach Winkelposition der Leiste 97 bezüglich des Stegs 95 ändern.

D.h. wird in den mit dem Bezugszeichen I versehenen Druckraum ein Fluid eingepresst und strömt eine entsprechende Fluidmenge aus dem mit dem Bezugszeichen II versehenen Druckraum aus, dreht der Rotationskolben 21 entsprechend den Volumenänderungen beider Druckräume I, II in Richtung des in Fig. 11 eingezeichneten Pfeils.

Der Druckaufbau oder -abbau in den in Fig. 11 dargestellten, Durchräumen I, II erfolgt vorzugsweise über eine nicht weiter dargestellte Handpumpe, die beispielsweise im Hohlschaft oder unmittelbar im Bereich der Betätigungsvorrichtung untergebracht ist und über die Betätigungsvorrichtung betrieben wird. D.h. die in Fig. 9 dargestellten Stangen 69 sind nicht an einen Kolben innerhalb der Instrumentenspitze, sondern beispielsweise an einen Kolben der Handpumpe angeschlossen, deren Pumpenkammern über Schläuche innerhalb des Hohlschafts an die Druckräume angeschlossen sind. Das Getriebe bzw. das Übersetzungsverhältnis zur Übersetzung einer Betätigungsbewegung der Betätigungsvorrichtung in eine Rotation des Rotationskolbens bestimmt sich dabei aus dem Volumenverhältnis der Druckkammern I, II zu den Pumpenkammern.

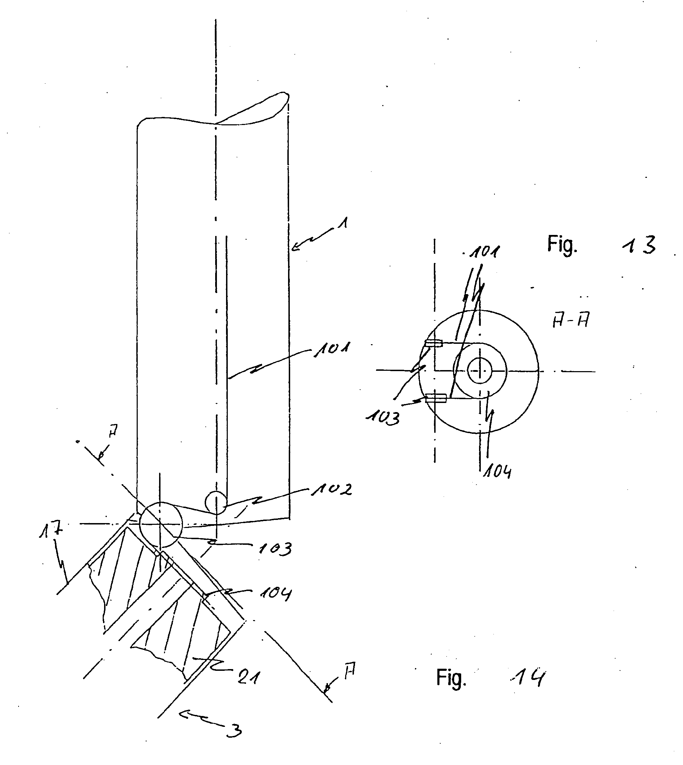

Schließlich zeigen die Fig. 12 und 13 ein viertes Ausführungsbeispiel der Erfindung, insbesondere den Scharnierbereich zwischen der Instrumentenspitze 3 und dem Hohlschaft 1.

Beim dritten Ausführungsbeispiel werden zwei Bowdenzüge 101 verwendet, die beidseits der Handhabe (in Fig. 12 nicht gezeigt) der Betätigungsvorrichtung angelenkt sind und vorzugsweise eine Handrotation um den Unterarm in eine zueinander gegenläufige Translationsbewegung umsetzen. Am distalen Endabschnitt des Hohlschafts 1 sind zwei koaxial zueinander ausgerichtete. Rollen 102 angeordnet, deren Laufrichtung der Längsachse des Hohlschafts folgt. Die gemeinsame Achse der Rollen 102 kreuzt dabei die Hohlschaftachse in einem rechten Winkel. Zur Hohlschaftachse radial versetzt sind zwei weitere Umlenkrollen 103 mit zu den Rollen 102 gleichen Laufrichtungen im Hohlschaft 1 gelagert, wobei die Umlenkrollen 103 vorzugsweise auf der Scharnierachse der Instrumentenspitze 3 und des Hohlschafts 1 sitzen.

Der in der Instrumentenspitze 3 bzw. der Hülse 17 gelagerte Rotationskolben 21 hat gemäß der Fig. 12 ebenfalls ein Laufrad 104, das fest an der proximalen Stirnseite des Kolbens 21 zentrisch befestigt ist. Die beiden Bowdenzüge 101 sind um die Rollen 102, die beiden Umlenkrollen 103 sowie das Laufrad 104 in dieser Reihenfolge geführt und an ihren Enden zu einem einzigen Zug miteinander verbunden. Durch diese Zugführung wird eine gegenläufige Translationsbewegung der Bowdenzüge 101 innerhalb des Hohlschafts 1 in eine Rotation des Rotationskolbens bzw. Rotationsbauteils 21 umgewandelt, wobei das Übersetzungsverhältnis zwischen der Betätigungsbewegung der Betätigungsvorrichtung und der Rotation des Rotationskolbens 21 und damit des Maulteils (nicht gezeigt) im wesentlichen durch den Durchmesser des Laufrads 104 bestimmt ist.

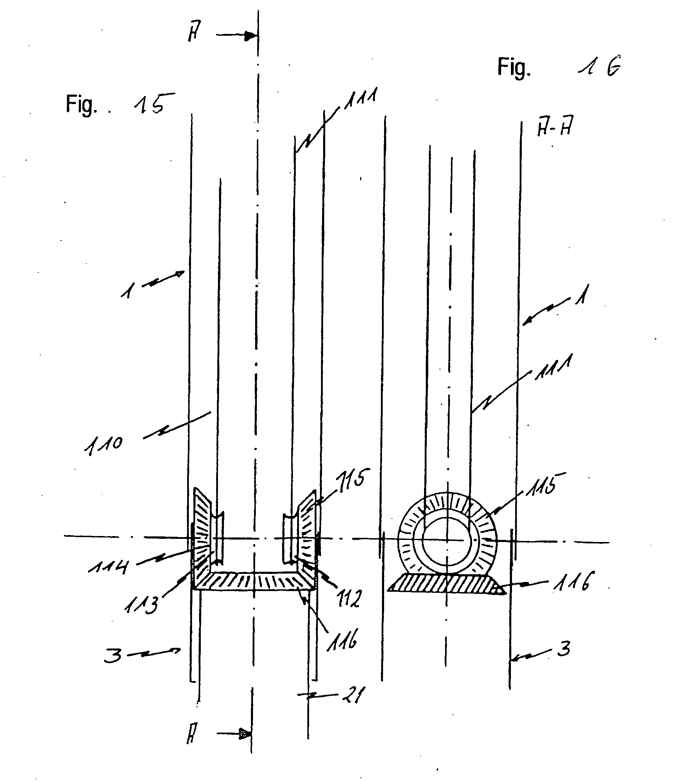

Als weitere Alternative wird in den Fig. 14 und 15 ein fünftes Ausführungsbeispiel für einen Moment- und Kraftübertragungsmechanismus gemäß der Erfindung im Anschlussbereich zwischen Rohrstück bzw. Hohlschaft 1 und Instrumentenspitze 3 dargestellt.

Demzufolge sind im Hohlschaft 1 Antriebsseile, Gummizüge oder Ketten 110, 111 geführt, die an ihren proximalen Enden an einer nicht weiter dargestellten Betätigungsvorrichtung angelenkt sind. An dieser Stelle sei darauf hingewiesen, dass bezüglich der Konstruktion einer Betätigungsvorrichtung vorstehend bereits zwei unterschiedliche Varianten vorgestellt wurden. Grundsätzlich ist es aber möglich die Betätigungsvorrichtung an den Moment- und Kraftübertragungsmechanismus anzupassen, solange die Ergonomie einer menschlichen Hand berücksichtigt wird.

Gemäß der Fig. 14 und 15 treiben die Antriebsseile oder Züge 110, 111 zwei sich gegenüberliegende co-axiale Reib- oder Kettenräder 112, 113 an, welche jeweils mit einem Kegelrad 114, 115 drehfest verbunden sind. Die gemeinsame Achsmittellinie der beiden separaten Räder 112, 113 samt deren Kegelräder 114, 115 schneidet dabei die Mittellinie des Rohrstücks bzw. Hohlschafts 1 in einem rechten Winkel. Dabei sind die Achsen der zwei Räderpaare am distalen Ende des Hohlschafts 1 montiert und dienen vorzugsweise auch als Anlenk- und Schwenkachsen für die Instrumentenspitze 3. In dem dem Hohlschaft 1 zugewandten Abschnitt der Instrumentenspitze 3, d.h. in der Aufnahme (nicht weiter dargestellt), ist ein weiteres Kegelrad 116 derart gelagert, dass seine Mittellinie co-axial zur Mittellinie der Aufnahme verläuft. Dieses weitere Kegelrad 116 ist drehfest mit dem Maulteil (in den Fig. 14 und 15 nicht dargestellt) bzw. dem Rotationsbauteil 21 verbunden und ist mit der beiden im Hohlschaft 1 gelagerten Kegelrädern 114, 115 in Kämmeingriff. Vorzugsweise hat das weitere Kegelrad 116 einen größeren Durchmesser als die beiden Kegelräder 114, 115 im Hohlschaft 1, wobei die Reib- oder Kettenräder 112, 113 wiederum einen kleiner Durchmesser als die zugehörigen Kegelräder 114, 115 haben.

Die Funktionsweise des Moment- und Kraftübertragungsmechanismus gemäß dem fünften Ausführungsbeispiel läst sich wie folgt erläutern:

Werden beide Reibräder 112, 113 in gleicher Drehrichtung mit der gleichen Drehgeschwindigkeit mittels der Züge 110, 111 angetrieben, wird über das Kegelrad 116 mit großem Durchmesser ein Schwenkmoment auf die Instrumentenspitze 3 übertragen, wodurch sich die Instrumentenspitze 3 mit den Radachsen als Schwenkpunkt bezüglich des Hohlschafts 1 abwinkelt. Durch die Dimensionierung des Kegelrads 116 mit großem Durchmesser bezüglich der Kegelräder 112, 113 mit kleinem Durchmesser bleiben die Kegelräder auch bei einem Schwenkwinkel von ca. 180° noch in Kämmeingriff, um das Schwenkmoment übertragen zu können. Vorzugsweise können die beiden Kegelräder 112, 113 mit kleinem Durchmesser auch noch elastisch auf deren Achsen gelagert sind, um den Kämmeingriff mit dem Kegelrad 116 gewährleisten zu können.

Soll nun in der derzeit eingenommenen Abwinkelposition das Maulteil in der Aufnahme gedreht werden, werden die beiden Reibräder 112, 113 aus ihrer derzeitigen Drehposition (relative Nullstellung) nunmehr gegenläufig jedoch mit gleichem Drehgeschwindigkeitsbetrag angetrieben, wobei sich das Kegelrad 116 mit großem Durchmesser um seine Mittelachse dreht und dabei das Maulteil rotiert.

## Patentansprüche

1. Chirurgisches Instrument mit einem Hohlschaft (1), an dessen einem, proximalen Ende eine Betätigungsvorrichtung (2) mit Handhabe (26, 27; 71), die ein knopfartiges Griffstück und eine Klinke (28) aufweist und an dessen anderem, distalen Ende eine zum Schaft abwinkelbare Instrumentenspitze (3) angeordnet ist, welche ein Maulteil (5) trägt das mittels dieser Klinke (28) geöffnet und geschlossen werden kann, sowie einem Getriebemechanismus, welcher zumindest eine erste Bewegung der Betätigungsvorrichtung (2), vorzugsweise auslösbar durch eine Handrotation einer Bedienperson, in eine Rotation des Maulteils (5) unter einem ersten bestimmten Übersetzungsverhältnis zur ersten Betätigungsbewegung transformiert,
**dadurch gekennzeichnet, dass**
die Betätigungsvorrichtung (2) einen Gelenkmechanismus umfasst, über den das Griffstück um seine Längsachse rotierbar für das Rotieren des Maulteils (5) um dessen Längsachse sowie abwinkelbar für das Abwinkeln der Instrumentenspitze (3) bezüglich des Hohlschafts (1) mit dem proximalen Ende des Hohlschafts (1) gekoppelt ist, wobei die Klinke (28) an dem Griffstück gelagert ist, um von der Bedienperson zusammen mit dem Griffstück betätigt werden zu können.

2. Chirurgisches Gerät nach Anspruch 1, **dadurch gekennzeichnet, dass**
die abwinkelbare Instrumentenspitze (3) eine hülsen- oder gehäuseförmige Aufnahme (4) zur drehbaren Lagerung des Maulteils (5) bildet, welche rotationsfest jedoch schwenkbar am distalen Ende des Hohlschafts (1) gelagert ist.

3. Chirurgisches Instrument nach Anspruch 2, **dadurch gekennzeichnet, dass**
der Getriebemechanismus ein Abwinkeln des Griffstücks, vorzugsweise auslösbar durch ein Handabwinkeln der Bedienerperson in ein Verschwenken der Aufnahme (4) bezüglich des Hohlschafts (1) unter einem zweiten bestimmten Übersetzungsverhältnis zur zweiten Betätigungsbewegung, transformiert.

4. Chirurgisches Instrument nach einem der vorstehenden Ansprüche, **dadurch gekennzeichnet, dass** der Gelenkmechanismus, am proximalen Ende des Hohlschafts (1) ausgebildet ist.

5. Chirurgisches Instrument nach Anspruch 4 in Verbindung mit Anspruch 3, **gekennzeichnet durch**
eine Aufnahme-Betätigungsstange (19; 66), die unmittelbar an der Handhabe (26, 27) in einem bestimmten Abstand zu deren Anlenkpunkt (31) am Gelenkmechanismus angelenkt, **durch** den Hohlschaft (1) geführt sowie an der Aufnahme (4) angelenkt ist und eine Schwenkbewegung der Handhabe (26, 27) in eine Abwinklung der Aufnahme (4) um deren Anlenkpunkt (18; 51)am distalen Hohlschaftende überträgt.

6. Chirurgisches Gerät nach einem der vorstehenden Ansprüche, **dadurch gekennzeichnet, dass**
der Getriebemechanismus eine torsionssteife biegsame Welle (24) umfasst, die in dem Hohlschaft (1) gelagert und mit dem Maulteil (5) für die Übertragung eines Rotationsmoments und damit eine Rotation des Maulteils in der Aufnahme (4) verbunden ist.

7. Chirurgisches Instrument nach Anspruch 6, **dadurch gekennzeichnet, dass**
an der Welle (24) ein Zahnradabschnitt (39) angeordnet ist, welcher über zumindest ein weiteres Zahnrad (40) unter Ausbildung eines Übersetzungsgetriebezugs in Kämmeingriff steht, das von der Betätigungsvorrichtung (2) antreibbar ist.

8. Chirurgisches Instrument nach Anspruch 7, **dadurch gekennzeichnet, dass**
der Gelenkmechanismus aus einem rotierbar in einem Lager (9) des Hohlschafts (1) aufgenommenen, das weitere Zahnrad (40) bildenden Hohlrad (41) besteht, welches den Zahnradabschnitt (39) der Welle (24) umgibt und einen Lagerkopf (29) für ein schwenkbares Anlenken der Handhabe (26, 27) bildet.

9. Chirurgisches Instrument nach einem der Ansprüche 6 bis 8, **dadurch gekennzeichnet, dass**
die Klinke (28) mit einem Maulteil-Betätigungszug (25) wirkverbunden ist, der innerhalb der biegsamen Welle' (24) geführt ist.

10. Chirurgisches Instrument nach Anspruch 9, **dadurch gekennzeichnet, dass**
eine Excenterstange (34) an der Klinke (28) angelenkt ist, über die ein erstes Ritzel (32) antreibbar ist, welches mit einem zweiten Ritzel (35) kämmt, das über eine exzentrisch daran gelagerte Verlängerungsstange (37) einen Kipphebel (38) betätigt, an den der Maulteilbetätigungszug (25) angelenkt ist, sodass die Bewegung der Verlängerungsstange (37) durch den Kipphebel (38) in die Bewegung des Maulteilbetätigungszugs (25) umgelenkt wird.

11. Chirurgisches Instrument nach einem der Ansprüche 1 bis 5, **dadurch gekennzeichnet, dass**
der Getriebemechanismus einen das Maulteil (5) tragenden Rotationskörper (52) umfasst, der drehbar in der Aufnahme (4) gelagert ist und mit einem eine axiale Spiralnut aufweisenden Kolben (57) in Gleiteingriff ist, der über die Betätigungsvorrichtung (2) verschiebbar ist, wobei die Schiebebewegung durch den Gleiteingriff des Rotationskörpers (52) in die Spiralnut in eine Rotationsbewegung des Rotationskörpers (52) und damit des Maulteils (5) transformiert wird.

12. Chirurgisches Instrument nach Anspruch 11, **dadurch gekennzeichnet, dass**
der Kolben (57) innerhalb des Rotationskörpers (52) gelagert ist und eine zentrale Axialbohrung hat in der eine an das Maulteil (5) gekoppelte Zug-/Druckstange (60) gelagert ist, die über eine Maulteil-Betätigungsstange (65) mit der Betätigungsvorrichtung (2) wirkverbunden ist.

## Claims

1. Surgical instrument with a hollow trunk (1) which has an actuating device (2) with a manipulator (26, 27; 71) comprising a knob-type grip piece and a "trigger" (28) arranged at its one, proximal, end, and an instrument tip (3) which can be angled with respect to the trunk and carries a jaw (5) that can be opened and closed by means of this "trigger" (28) arranged at its other, distal, end, and with a gear mechanism which converts at least a first motion of the actuating device (2), preferably imparted by a rotation of the operator's hand, into a rotation of the jaw (5) at a first specific transmission ratio to the first actuating motion,
**characterized in that**
the actuating device (2) comprises a joint mechanism which couples the grip piece to the proximal end of the hollow trunk (1), rotatably about the grip piece's longitudinal axis for rotation of the jaw (5) about the jaw's longitudinal axis, and also "tiltably" for angling the instrument tip (3) with respect to the hollow trunk (1), the "trigger" (28) being mounted on the grip piece so that it can be actuated by the operator together with the grip piece.

2. Surgical instrument according to Claim 1, **characterized in that**
the bendable instrument tip (3) forms a sleeve-like or casing-like holder (4), mounted fixedly in rotation but pivotably at the distal end of the hollow trunk (1), for the rotatable mounting of the jaw (5).

3. Surgical instrument according to Claim 2, **characterized in that**
the gear mechanism converts an angling of the grip piece, preferably imparted by a bend of the operator's hand, into a tilting of the holder (4) with respect to the hollow trunk (1) at a second specific transmission ratio to the second actuating motion.

4. Surgical instrument according to any one of the preceding claims, **characterized in that**
the joint mechanism is constructed at the proximal end of the hollow trunk (1).

5. Surgical instrument according to Claim 4 in conjunction with Claim 3, **characterized by**
a holder actuating rod (19; 66) which is linked directly to the manipulator (26, 27) at a specific distance from the latter's hinge point (31), is led through the hollow trunk (1), and is linked to the holder (4), and converts a pivoting motion of the manipulator (26, 27) into an angling of the holder (4) about its hinge point (18; 51) at the distal end of the hollow trunk.

6. Surgical instrument according to any one of the preceding claims, **characterized in that**
the gear mechanism comprises a torsionally stiff but flexible shaft (24) which is carried inside the hollow trunk (1) and is connected to the jaw (5) for transmission of rotational torque and thus rotation of the jaw in the holder (4).

7. Surgical instrument according to Claim 6, **characterized in that**
a toothed gear section (39) arranged on the shaft (24) meshes with at least one further gear (40) to form a step-up gear train which can be driven by the actuating device (2).

8. Surgical instrument according to Claim 7, **characterized in that**
the joint mechanism consists of a a hollow gear (41) forming the "further gear" (40), which is seated rotatably in a bearing (9) of the hollow trunk (1), surrounds the toothed gear section (39) of the shaft (24), and forms a bearing head (29) for the pivotable linkage of the manipulator (26, 27).

9. Surgical instrument according to any one of Claims 6 to 8, **characterized in that**
the "trigger" (28) is actively connected to a jaw-actuating cable (25) routed inside the flexible shaft (24).

10. Surgical instrument according to Claim 9, **characterized in that**
an eccentric rod (34) linked to the "trigger" (28) is able to drive a first pinion (32) meshing with a second pinion (35) which, through an extension rod (37) eccentrically mounted thereon, actuates a rocker (38) to which the jaw-actuating cable (25) is linked, so that the motion of the extension rod (37) is transposed by the rocker (38) into a motion of the jaw-actuating cable (25).

11. Surgical instrument according to any one of Claims 1 to 5, **characterized in that**
the gear mechanism comprises a body of revolution (52) which carries the jaw (5) and is mounted rotatably in the holder (4) and is in sliding engagement with a plunger (57) that has an axial helical groove and is displaceable by the actuating device (2), the translational motion being converted by the sliding engagement of the body of revolution (52) in the helical groove into a rotational motion of the body of revolution (52) and thus of the jaw (5).

12. Surgical instrument according to Claim 11, **characterized in that**
the plunger (57) is mounted inside the body of revolution (52), and has a central axial bore in which a tension/compression rod (60), which is coupled to the jaw (5) and is actively connected to the actuating device (2) by a jaw-actuating rod (65), is carried.

## Revendications

1. Instrument chirurgical avec une tige creuse (1), à une première extrémité proximale de laquelle est disposé un dispositif d'actionnement (2) muni d'une manette (26, 27 ; 71) qui présente une pièce de saisie du genre d'un bouton et un cliquet (28) et, à son autre extrémité distale, une pointe d'instrument (3) pouvant être inclinée sous un certain angle par rapport à la tige, pointe portant une partie de bec (5) qui peut être ouverte et fermée au moyen de ce cliquet (28), ainsi qu'un mécanisme de transmission transformant au moins un premier déplacement du dispositif d'actionnement (2), de préférence pouvant être déclenché par une rotation manuelle d'un opérateur, en une rotation de la partie de bec (5) dans un premier rapport de transmission, déterminé par rapport au premier déplacement d'actionnement, **caractérisé en ce que** le dispositif d'actionnement (2) comprend un mécanisme d'articulation, par lequel la pièce de saisie peut être couplée avec l'extrémité proximale de la tige creuse (1), en pouvant tourner autour son axe longitudinal, pour faire tourner la partie de bec (5) autour de son axe longitudinal, ainsi que en pouvant subir un changement d'orientation angulaire pour dévier angulairement la pointe d'instrument (3) par rapport à la tige creuse (1), le cliquet (28) étant monté sur la pièce de saisie et pouvant être actionné par l'opérateur, conjointement avec la pièce de saisie.

2. Instrument chirurgical selon la revendication 1, **caractérisé en ce que** la pointe d'instrument (3), pouvant être déviée angulairement forme un logement (4), en forme de douille ou de boîtier, pour permettre un montage en palier rotatif de la partie de bec, logement monté de façon bloquée en rotation, en pouvant cependant pivoter sur l'extrémité distale de la tige creuse (1).

3. Instrument chirurgical selon la revendication 2, **caractérisé en ce que** le mécanisme de transmission transforme un changement d'orientation angulaire de la pièce de saisie, de préférence pouvant être déclenché par un changement d'orientation angulaire manuel de l'opérateur, en un pivotement du logement (4) par rapport à la tige creuse (1), avec un deuxième rapport de transmission, déterminé, par rapport au deuxième déplacement d'actionnement.

4. Instrument chirurgical selon l'une des revendications précédentes, **caractérisé en ce que** le mécanisme d'articulation est réalisé sur l'extrémité proximale de la tige creuse (1).

5. Instrument chirurgical selon la revendication 4, en liaison avec la revendication 3, **caractérisé par** une tige d'actionnement de logement (19 ; 66), articulée directement sur la manette (26, 27) selon un espacement déterminé par rapport à son point d'articulation (31), en étant guidée par la tige creuse (1) et articulée sur le logement (4), et transmettant un mouvement de pivotement de la manette (26, 27) en un changement d'orientation angulaire du logement (4) autour de son point d'articulation (18 ; 51), à l'extrémité distale de la tige creuse.

6. Instrument chirurgical selon l'une des revendications précédentes, **caractérisé en ce que** le mécanisme de transmission comprend un arbre (24) flexible, rigide en torsion, monté dans la tige creuse (1) et relié à la partie de bec (5) pour la transmission d'un mouvement de rotation et, ainsi, provoquer une rotation de la partie de bec dans le logement (4).

7. Instrument chirurgical selon la revendication 6, **caractérisé en ce que**, sur l'arbre (24), est disposé un tronçon de roue dentée (39) qui est en engrènement de denture, par l'intermédiaire d'au moins une autre roue dentée (40), en formant un train de transmission à démultiplication, pouvant être entrainé par le dispositif d'actionnement (2).

8. Instrument chirurgical selon la revendication 7, **caractérisé en ce que** le mécanisme d'articulation est composé d'une roue creuse (41), supportée à rotation dans un palier (9) de la tige creuse (1) et formant l'autre roue dentée (40), roue creuse qui entoure le tronçon de roue dentée (39) de l'arbre (24) et forme une tête de palier (29) pour une articulation pivotante de la manette (26, 27).

9. Instrument chirurgical selon l'une des revendications 6 à 8, **caractérisé en ce que** le cliquet (28) est relié fonctionnellement au train d'actionnement de partie de bec (25) guidé à l'intérieur de l'arbre (24) flexible.

10. Instrument chirurgical selon la revendication 9, **caractérisé en ce que**, sur le cliquet (28) est articulée une tige d'excentrique (34), par l'intermédiaire de laquelle peut être entrainé un premier pignon (32), s'engrenant avec un deuxième pignon (35) qui, par l'intermédiaire d'une tige de prolongement (37) montée sur celui-ci, actionne un levier basculant (38) sur lequel est articulé le train d'actionnement de partie de bec (25), de manière que le déplacement de la tige de prolongement (37) soit transformé, par le levier basculant (38), en le déplacement du train d'actionnement de partie de bec (25).

11. Instrument chirurgical selon l'une des revendications 1 à 5, **caractérisé en ce que** le mécanisme de transmission comprend un corps de rotation (52), portant la partie de bec (5), monté à rotation dans le logement (4) et mis en prise par glissement avec un piston (57) présentant une gorge spirale axiale, piston déplaçable par l'intermédiaire du dispositif d'actionnement (2), le déplacement de coulissement étant transformé, par la mise en prise avec glissement du corps de rotation (52) dans la gorge spirale, en un mouvement de rotation du corps de rotation (52) et, ainsi, de la partie de bec (5).

12. Instrument chirurgical selon la revendication 11, **caractérisé en ce que** le piston (57) est monté à l'intérieur du corps de rotation (52) et présente un perçage axial central, dans lequel est montée une tige de traction/compression (60), couplée à la partie de bec (5) et reliée fonctionnellement au dispositif d'actionnement (2), par l'intermédiaire d'une tige d'actionnement de partie de bec (65).
